# EUROPÄISCHE PATENTSCHRIFT

(11) **EP 2 848 250 B1**
(45) Veröffentlichungstag und Bekanntmachung des Hinweises auf die Patenterteilung: **07.09.2016**
(21) Anmeldenummer: 13787743.7
(22) Anmeldetag: 26.04.2013
(51) Int. Cl.: A61P 19/08, A61K 33/14, A61K 33/24, A61L 24/00, A61K 31/663, A61L 24/02, A61K 9/00, A61K 47/02, A61K 9/08

(54) **VERFAHREN ZUR REGENERATION VON KNOCHENGEWEBE**
METHOD FOR REGENERATING BONE TISSUE
MÉTHODE DE RÉGÉNÉRATION D'UN TISSU OSSEUX

(30) Priorität: 10.05.2012 RU 2012119192
(43) Veröffentlichungstag der Anmeldung: 18.03.2015
(73) Patentinhaber: FELIX ltd., Respublika Tatarstan, 423834 (RU)
(72) Erfinder: DEVYATOV, Fedor Vladimirovich, 420025 Kazan (RU)
(74) Vertreter: Jeck, Anton
(86) Internationale Anmeldenummer: PCT/RU2013/000366
(87) Internationale Veröffentlichungsnummer: WO 2013/169146

(56) Entgegenhaltungen:
- RU-C1- 2 248 210
- RU-C1- 2 360 663
- RU-C1- 2 386 437
- US-A1- 2005 147 645

## Beschreibung

Die Erfindung betrifft ein Verfahren zur Knochengeweberegeneration nach dem Oberbegriff des Anspruchs.

Die Erfindung ist bei der Behandlung von unterschiedlichen Knochenverletzungen, insbesondere Brüchen und Fissuren, einsetzbar. Sie kann bei der medizinischen und tierärztlichen Therapie und Chirurgie realisiert werden.

Aus dem Stand der Technik ist ein Verfahren zur Knochengeweberegeneration im Rahmen eines Versuchs bekannt, in dem Bruchstücke eines verletzten Knochens mit einem Gipsverband immobilisiert werden. Dabei wird eine Wasserlösung mit Etidronsäure (1-Hydroxyethyliden-diphosphonsäure *(1-Hydroxyethan-1,1-diphosphonsäure (HEDP),* Calciumchlorid und Gadoliniumnitrat (III) bei Raumtemperatur in den Bruchbereich eingeführt [1]. Der Mangel dieses bekannten Verfahrens ist ein verhältnismäßig langer Prozess einer Knochengeweberegeneration.

Der nächstkommende Stand der Technik gegenüber der Erfindung ist ein Verfahren zur Knochengeweberegeneration im Rahmen eines Versuchs gemäß [2]. Nach diesem Verfahren werden Bruchstücke eines verletzten Knochens mit einem Gipsverband oder einer Kunststoff-Binde immobilisiert. Danach wird eine Wasserlösung in den Bruchbereich eingeführt. Die Wasserlösung enthält Etidronsäure (1-Hydroxyethyliden-diphosphonsäure) in einer Menge von (1,80 - 2,06) g/l, wasserfreies Calciumchlorid in einer Menge von (1,44 - 2,22) g/l, Gadoliniumnitrat (III) Hexahydrat in einer Menge von (0,30 - 0,40) g/l und Dysprosiumchlorid (III) Hexahydrat in einer Menge von (0,038 - 0,076) g/l mit pH (7,3 - 7,8) bei Raumtemperatur. Dieses bekannte Verfahren wird als Prototyp angesehen und ist ebenfalls dadurch bemängelt, dass die Knochengeweberegeneration verhältnismäßig lange dauert.

Es ist Aufgabe der Erfindung, eine zusätzliche Verkürzung der Dauer von Knochengeweberegeneration an der Stelle der Knochenverletzung oder des Knochendefekts zu erreichen, bis die normale physiologische Funktion des verletzten/betroffenen Knochens wieder hergestellt ist.

Die gestellte Aufgabe wird durch die Merkmale des Anspruchs gelöst.

Das wird wie folgt erreicht.

Gemäß dem bekannten Verfahren zur Knochengeweberegeneration im Rahmen eines Versuchs werden Bruchstücke eines verletzten Knochens mit einem Gipsverband oder einer Kunststoff-Binde immobilisiert. Danach wird eine Wasserlösung in den Bruchbereich eingeführt. Die Wasserlösung enthält dabei Etidronsäure (1-Hydroxyethyliden-Diphosphonsäure) in einer Menge von (1,80 - 2,06) g/l, wasserfreies Calciumchlorid in einer Menge von (1,44 - 2,22) g/l, Gadoliniumnitrat (III) Hexahydrat in einer Menge von (0,30 - 0,40) g/l, Dysprosiumchlorid (III) Hexahydrat in einer Menge von (0,038 - 0,076) g/l, mit pH (7,3 - 7,8). Bevor die genannte Wasserlösung in den Bruchbereich eingeführt wird, wird sie zuerst erfindungsgemäß bis zu einer Temperatur von (30 - 100)°C geführt, bei dieser Temperatur im Laufe von (1 - 48) Stunden gehalten und dann wieder auf Raumtemperatur abgekühlt.

Infolge der Anwendung des Verfahrens wird die Knochengeweberegeneration wesentlich beschleunigt. Die Erholungszeit, bis die normale physiologische Funktion des verletzten/betroffenen Knochens wiederhergestellt ist, kann im Vergleich mit der Zeit nach dem Prototyp-Verfahren [2] zusätzlich um 15 bis 20 % verkürzt werden.

Bis jetzt wurde in der Fachliteratur kein Verfahren zur Knochengeweberegeneration im Rahmen eines Versuchs beschrieben, welches der Gesamtheit seiner Merkmale nach mit dem hier beanspruchten und beschriebenen Verfahren völlig identisch wäre. Das oben Dargelegte gibt den Erfindern den Anlass zu behaupten, dass der hier beanspruchte Gegenstand neu ist. Die Gegenüberstellung der bekannten Merkmale nach dem Prototyp-Verfahren [2] und der Merkmale seiner vorgenommenen Modifikationen, und zwar die Vorerwärmung der verwendeten Lösung bis zu einer oben genannten genauen Temperatur (30 - 100)° C und das Halten bei dieser Temperatur im Laufe von einer genau definierten Zeit (1 - 48 Stunden), lässt die im Rahmen eines Versuchs beobachtete wesentliche Verkürzung der Erholungszeit, bis die normale physiologische Funktion des verletzten/betroffenen Knochens wiederhergestellt ist, im Vergleich zur Zeitangabe nach dem aus dem Stand der Technik bekannten Verfahren [2] nicht vorhersagen. Das oben Dargelegte gibt Anlass zu behaupten, dass die hier beanspruchte technische Lösung hinsichtlich des bekannten Stands der Technik nicht naheliegend ist und folglich auf erfinderischer Tätigkeit beruht. Das erfindungsgemäße Verfahren bedarf keiner speziellen Ausrüstung und kann mühelos sogar unter ambulanten Bedingungen ausgeführt werden. Folglich wird auch die dritte Voraussetzung der Patentierbarkeit - gewerbliche Anwendbarkeit - erfüllt.

Das angemeldete Verfahren zur Kochengeweberegeneration kann anhand folgender Beispiele näher erklärt werden.

### Beispiel 1 (Zubereitung der angemeldeten Lösung)

In ein 1000 ml großes Messglas wird Etidronsäure (1-Hydroxyethyliden-Diphosphonsäure) in einer Menge von (1,80 - 2,06) g, wasserfreies Calciumchlorid in einer Menge von (1,44 - 2,22) g, Gadoliniumnitrat (III) Hexahydrat in einer Menge von (0,30 - 0,40) g und Dysprosiumchlorid (III) Hexahydrat in einer Menge von (0,038 - 0,076) g sowie 950 ml destilliertes Wasser gefüllt. Die gebildete Mischung wird mit einer konzentrierten Lösung von einem alkalischen Agens (z. B. 10%iger Wasserlösung von Natriumhydroxid) auf einen pH-Wert von 7,3 - 7,8 gebracht. Die resultierende Lösung wird mit destilliertem Wasser auf einen Inhalt von 1000 ml gebracht. Danach wird die Lösung bis auf 30 - 100° C erwärmt, bei dieser Temperatur innerhalb von 1 bis 48 Stunden gehalten und dann bis auf die Raumtemperatur von 20 - 25° C abgekühlt. Die hergestellte Lösung wird nachher für die Ausführung des erfindungsgemäßen Verfahrens benutzt.

### Beispiel 2

Eine Wasserlösung mit folgender Zusammensetzung in g/l wird zubereitet:

| | |
|---|---|
| Etidronsäure (1-Hydroxyethyliden-Diphosphonsäure) | 1,80 |
| wasserfreies Calciumchlorid | 1,44 |
| Gadoliniumnitrat (III) Hexahydrat | 0,30 |
| Dysprosiumchlorid (III) Hexahydrat | 0,038 |

und hat einen pH-Wert 7,3. Diese Wasserlösung wird danach auf 30° C erwärmt und bei dieser Temperatur innerhalb von 48 Stunden gehalten. Dann wird die Lösung bis auf die Raumtemperatur abgekühlt. Die auf diese Weise zubereitete Lösung wurde einer betäubten Hauskatze (nordeuropäische Rasse) eingespritzt. Die Hauskatze bekam vorher eine auf künstliche Weise gemachte Fraktur von Unterschenkelknochen des Hinterbeins (der hinteren Extremität). Die erfindungsgemäße Lösung in einer Menge von 2,0 ml wurde 1 Stunde nach dem Beibringen der Verletzung innerhalb von 2 Min. an der Stelle der Knochenverletzung verabreicht. Nach der Injektion werden die Knochenbruchstellen mit einem festen Gipsverband versehen oder mit einer Kunststoff-Binde umspannt. Die verletzten Segmente der Extremität verbleiben in diesem Verband, bis die Stütz- und Bewegungsfunktion wieder hergestellt ist. Der Regenerationsablauf des verletzten Knochengewebes wird röntgenographisch verfolgt. Dafür wird eine röntgendurchlässige synthetische Binde CELLACAST XTRA, Fabrikat Lohmaim & Rausher eingesetzt. Alternativ können an der Verletzungsstelle spezielle "Fenster" im Gipsverband ausgeschnitten werden. Der Zeitpunkt der Knochengeweberegeneration wird visuell am Verhalten des betroffenen Tieres bestimmt (ab dem Zeitpunkt der Lösungseinspritzung bis zu einem Zeitpunkt von +0,5 Tagen, wo die Stütz- und Bewegungsfunktion der verletzten Extremität völlig wieder hergestellt ist). Diese Angabe ist für den hier betrachteten Fall der Tabelle 1 zu entnehmen.

### Beispiel 3

Der Ablauf ist mit der allgemeinen Vorgehensweise nach dem Beispiel 2 identisch, jedoch wird zur Einspritzung eine Wasserlösung in einer Menge von 2,0 ml in folgender Zusammensetzung in g/l verwendet:

| | |
|---|---|
| Etidronsäure (1-Hydroxyethyliden-Diphosphonsäure) | 1,92 |
| wasserfreies Calciumchlorid | 1,88 |
| Gadoliniumnitrat (III) Hexahydrat | 0,35 |
| Dysprosiumchlorid (III) Hexahydrat | 0,055 |

mit einem pH-Wert 7,5. Diese Lösung wird nachher bis auf 60° C erwärmt und bei dieser Temperatur innerhalb von 6 Stunden gehalten. Die Knochengeweberegenerationsdauer für diesen Fall ist der Tabelle 1 zu entnehmen.

### Beispiel 4

Die Vorgehensweise ist mit der nach dem Beispiel 2 identisch, jedoch wird zur Einspritzung eine Wasserlösung in einer Menge von 2,0 ml in folgender Zusammensetzung in g/lverwendet:

| | |
|---|---|
| Etidronsäure (1-Hydroxyethyliden-Diphosphonsäure) | 1,92 |
| wasserfreies Calciumchlorid | 1,88 |
| Gadoliniumnitrat (III) Hexahydrat | 0,35 |
| Dysprosiumchlorid (III) Hexahydrat | 0,055 |

mit einem pH-Wert 7,7. Diese Lösung wird nachher bis auf 70° C erwärmt und bei dieser Temperatur innerhalb von 4 Stunden gehalten. Die Knochengeweberegenerationsdauer für diesen Fall ist der Tabelle 1 zu entnehmen.

### Beispiel 5

Der Ablauf ist mit der allgemeinen Vorgehensweise nach dem Beispiel 2 identisch, jedoch wird zur Einspritzung eine Wasserlösung in einer Menge von 2,0 ml in folgender Zusammensetzung in g/l verwendet:

| | |
|---|---|
| Etidronsäure (1-Hydroxyethyliden-Diphosphonsäure) | 2,06 |
| wasserfreies Calciumchlorid | 2,20 |
| Gadoliniumnitrat (III) Hexahydrat | 0,40 |
| Dysprosiumchlorid (III) Hexahydrat | 0,076 |

mit einem pH-Wert 7,8. Diese Lösung wird nachher bis auf 100° C erwärmt und bei dieser Temperatur innerhalb von 1 Stunde gehalten.

### Beispiel 6

Die Vorgehensweise ist mit der nach dem Beispiel 2 identisch, jedoch wird zur Einspritzung eine Wasserlösung in einer Menge von 2,0 ml in folgender Zusammensetzung in g/l verwendet:

| | |
|---|---|
| Etidronsäure (1-Hydroxyethyliden-Diphosphonsäure) | 1,80 |
| wasserfreies Calciumchlorid | 1,44 |
| Gadoliniumnitrat (III) Hexahydrat | 0,30 |
| Dysprosiumchlorid (III) Hexahydrat | 0,038 |

mit einem pH-Wert 7,5. Diese Lösung wird nachher bis auf 70° C erwärmt und bei dieser Temperatur innerhalb von 4 Stunden gehalten. Die Knochengeweberegenerationsdauer für diesen Fall ist der Tabelle 1 zu entnehmen.

### Beispiel 7

Die Vorgehensweise ist mit der nach dem Beispiel 2 identisch, jedoch wird zur Einspritzung eine Wasserlösung in einer Menge von 2,0 ml in folgender Zusammensetzung in g/l verwendet:

| | |
|---|---|
| Etidronsäure (1-Hydroxyethyliden-Diphosphonsäure) | 1,80 |
| wasserfreies Calciumchlorid | 1,44 |
| Gadoliniumnitrat (III) Hexahydrat | 0,30 |
| Dysprosiumchlorid (III) Hexahydrat | 0,038 |

mit einem pH-Wert 7,8. Diese Lösung wird nachher bis auf 70° C erwärmt und bei dieser Temperatur innerhalb von 4 Stunden gehalten. Die Knochengeweberegenerationsdauer für diesen Fall ist der Tabelle 1 zu entnehmen.

### Beispiel 8 (Vergleich)

Die Vorgehensweise ist mit der nach dem Beispiel 3 identisch, jedoch wird der pH-Wert der nach diesem Beispiel benutzten Lösung gleich 7,0 gesetzt. Die Knochengeweberegenerationsdauer für diesen Fall ist der Tabelle 1 zu entnehmen.

### Beispiel 9 (Vergleichsbeispiel)

Die Durchführung ist wie in dem Beispiel 3, jedoch wird der pH-Wert der nach diesem Beispiel benutzten Lösung gleich 8,0 gesetzt. Die Knochengeweberegenerationsdauer für diesen Fall ist der Tabelle 1 zu entnehmen.

### Beispiel 10 (Vergleichsbeispiel)

Die Vorgehensweise ist mit der nach dem Beispiel 5 identisch, aber das Halten bei der für dieses Verfahren angegebenen Temperatur erfolgt im Laufe von 0,5 Stunden. Die Knochengeweberegenerationsdauer für diesen Fall ist der Tabelle 1 zu entnemen.

### Beispiel 11 (Vergleichsbeispiel)

Die Vorgehensweise ist mit der nach dem Beispiel 2 identisch, aber das Halten bei der für dieses Verfahren angegebenen Temperatur erfolgt im Laufe von 60 Stunden. Die Knochengeweberegenerationsdauer für diesen Fall ist der Tabelle 1 zu entnehmen.

### Beispiel 12 (gemäß dem Stand der Technik [1])

Die Vorgehensweise ist mit dem allgemeinen Beispiel 2 identisch, jedoch wird zur Einspritzung eine Wasserlösung in einer Menge von 2,0 ml in folgender Zusammensetzung in g/l verwendet:

| | |
|---|---|
| Etidronsäure (1-Hydroxyethyliden-Diphosphonsäure) | 2,00 |
| wasserfreies Calciumchlorid | 2,20 |
| Gadoliniumnitrat (III) Hexahydrat | 0,40 |

mit einem pH-Wert 8,0. Solche Schritte, wie eine Erwärmung der Lösung bis auf 30° C und Halten bei dieser Temperatur, werden ausgeschlossen. Die Knochengeweberegenerationsdauer für diesen Fall ist in der Tabelle 1 angegeben.

### Beispiel 13 (nach Prototyp [2])

Die Vorgehensweise ist mit der nach dem Beispiel 4 identisch. Dabei werden die darin genannte Zusammensetzung der Lösung und der entsprechende pH-Wert benutzt, aber die Schritte der Erwärmung der Lösung bis zur Temperatur von 70° C und des Haltens bei dieser Temperatur werden ausgeschlossen. Die Knochengeweberegenerationsdauer für diesen Fall ist der Tabelle 1 zu entnehmen.

### Beispiel 14 (nach Prototyp [2])

Die Vorgehensweise ist mit der nach dem Beispiel 4 identisch. Dabei werden die darin genannte Zusammensetzung der Lösung und der entsprechende pH-Wert benutzt. Aber die Schritte der Erwärmung der Lösung bis 70° C und des Haltens bei dieser Temperatur werden ausgeschlossen. Die Knochengeweberegenerationsdauer für diesen Fall ist in der Tabelle 1 angegeben.

### Beispiel 15 (nach Prototyp [2])

Die Vorgehensweise ist mit der nach dem Beispiel 5 identisch. Dabei werden die darin genannte Zusammensetzung der Lösung und der entsprechende pH-Wert benutzt. Aber die Schritte der Erwärmung der Lösung bis 100° C und des Haltens bei dieser Temperatur werden ausgeschlossen. Die Knochengeweberegenerationsdauer für diesen Fall ist in der Tabelle 1 angegeben.

**Tabelle 1**

| Beispiel Nr. | Verletztes Tier | Zeit bis zur vollständigen Instandsetzung der Stütz- und Bewegungsfunktion des verletzten Knochensegments, Tage |
|---|---|---|
| 2 | Katze, nordeuropäische Rasse | 5,0 |
| 3 | Katze, nordeuropäische Rasse | 4,5 |
| 4 | Katze, nordeuropäische Rasse | 4,5 |
| 5 | Katze, nordeuropäische Rasse | 5,0 |
| 6 | Katze, nordeuropäische Rasse | 4,5 |
| 7 | Katze, nordeuropäische Rasse | 5,0 |
| 8 (Vergleichsbeispiel) | Katze, nordeuropäische Rasse | 6,0 |
| 9 (Vergleichsbeispiel) | Katze, nordeuropäische Rasse | 6,5 |
| 10 (Vergleichsbeispiel) | Katze, nordeuropäische Rasse | 6,0 |
| 11 (Vergleichsbeispiel) | Katze, nordeuropäische Rasse | 4,5 |
| 12 (Stand der Technik) | Katze, nordeuropäische Rasse | 9,0 |
| 13 (Prototyp) | Katze, nordeuropäische Rasse | 6,0 |
| 14 (Prototyp) | Katze, nordeuropäische Rasse | 6,5 |
| 15 (Prototyp) | Katze, nordeuropäische Rasse | 6,0 |

### Beispiel 16

Eine Wasserlösung mit folgender Zusammensetzung in g/l wird zubereitet:

| | |
|---|---|
| Etidronsäure (1-Hydroxyethyliden-Diphosphonsäure) | 1,80 |
| wasserfreies Calciumchlorid | 1,44 |
| Gadoliniumnitrat (III) Hexahydrat | 0,30 |
| Dysprosiumchlorid (III) Hexahydrat | 0,038 |

mit einem pH-Wert 7,3. Danach wird die Lösung bis zur Temperatur von 30° C erwärmt und bei dieser Temperatur innerhalb von 48 Stunden gehalten. Dann wird die Lösung bis zur Raumtemperatur abgekühlt. Diese Lösung wird einem betäubten Kaninchen unbestimmter Rasse eingespritzt. Das Kaninchen wird gezielt künstlich verletzt, indem beide Oberschenkelknochen unter Vollnarkose mit einer elektrischen Bohrmaschine mit einem Bohrerdurchmesser von 5 mm gebohrt werden. Dabei wird die o. g. Lösung in einer Menge von 1,0 ml (die Injektionszeit beträgt 2 Minuten wie im Beispiel 2) an der verletzten Stelle eines der betroffenen Knochen eingespritzt (der dritte ähnliche Knochen wird als Referenzknochen benutzt). Danach werden alle verletzten Stellen mit einem festen Gipsverband versehen oder mit einer Kunststoff-Binde umwickelt. Der Prozess der Knochengeweberegeneration wird röntgenographisch, wie im Beispiel 2 beschrieben, überwacht. Der Zeitpunkt der Knochengeweberegeneration wird visuell nach dem Verhalten des verletzten Tieres bestimmt (ab dem Zeitpunkt der Lösungseinspritzung bis zum Zeitpunkt (bis auf +0,5 Tage), wo die Stütz- und Bewegungsfunktion der verletzten Extremität völlig wiederhergestellt ist). Dieser Kennwert ist für diesen spezifischen Fall der Tabelle 2 zu entnehmen.

### Beispiel 17

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 3 beschrieben ist.

### Beispiel 18

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 4 beschrieben ist.

### Beispiel 19

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 5 beschrieben ist.

### Beispiel 20

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 6 beschrieben ist.

### Beispiel 21

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 7 beschrieben ist.

### Beispiel 22 (Vergleichsbeispiel)

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 8 beschrieben ist.

### Beispiel 23 (Vergleichsbeispiel)

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 9 beschrieben ist.

### Beispiel 24 (Vergleichsbeispiel)

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 10 beschrieben ist.

### Beispiel 25 (Vergleichsbeispiel)

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 11 beschrieben ist.

### Beispiel 26 (gemäß dem Stand der Technik [1])

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 12 beschrieben ist.

### Beispiel 27 (nach Prototyp [2])

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 13 beschrieben ist.

### Beispiel 28 (nach Prototyp [2])

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 14 beschrieben ist.

### Beispiel 29 (nach Prototyp [2])

Die Vorgehensweise ist mit dem allgemeinen Beispiel 16 identisch, aber als Injektionslösung wird eine Wasserlösung verwendet, deren Zusammensetzung und Zubereitungstechnik vor der Injektion im Beispiel 15 beschrieben ist.

Die Vergleichsangaben hinsichtlich der Knochengeweberegenerationsdauer sind in der Tabelle 2 enthalten. Dabei handelt es sich um die Regenerationsdauer des Knochengewebes, welches vor der Heilung mit den in den Beispielen 16 bis 29 genannten Lösungen mittels Injektion behandelt wurde, und um die Regenerationsdauer des Knochengewebes, welches mit keiner Injektion behandelt wurde.

**Tabelle 2**

| Beispiel Nr. | Verletztes Tier | Zeit bis zur vollständigen Instandsetzung der Stütz- und Bewegungsfunktion des verletzten Knochensegments, Tage | |
|---|---|---|---|
| | | für Knochen mit Injektion der Lösung | für Referenzknochen |
| 16 | Kaninchen unbestimmter Rasse | 4,0 | 18,0 |
| 17 | Kaninchen unbestimmter Rasse | 3,5 | 17,5 |
| 18 | Kaninchen unbestimmter Rasse | 4,0 | 18,0 |
| 19 | Kaninchen unbestimmter Rasse | 4,0 | 18,0 |
| 20 | Kaninchen unbestimmter Rasse | 4,0 | 17,5 |
| 21 | Kaninchen unbestimmter Rasse | 3,5 | 18,0 |
| 22 (Vergleichsbeispiel) | Kaninchen unbestimmter Rasse | 4,5 | 18,0 |
| 23 (Vergleichsbeispiel) | Kaninchen unbestimmter Rasse | 4,5 | 17,5 |
| 24 (Vergleichsbeispiel) | Kaninchen unbestimmter Rasse | 4,5 | 17,5 |
| 25 (Vergleichsbeispiel) | Kaninchen unbestimmter Rasse | 4,0 | 18,0 |
| 26 (Stand der Technik) | Kaninchen unbestimmter Rasse | 8,5 | 18,0 |
| 27 (Prototyp) | Kaninchen unbestimmter Rasse | 5,0 | 17,5 |
| 28 (Prototyp) | Kaninchen unbestimmter Rasse | 5,0 | 18,0 |
| 29 (Prototyp) | Kaninchen unbestimmter Rasse | 5,5 | 18,5 |

### Beispiel 30

Es wird eine Wasserlösung hergestellt, deren Zusammensetzung und Zubereitungsverfahren im Beispiel 3 beschrieben sind. Diese Lösung wird einem betäubten Hofhund unbestimmter Rasse eingespritzt. Der Hund bekommt künstlich eine Fraktur des Oberarmbeins der rechten Vorderpfote mit einer starken Verschiebung. 6 Stunden nach dem Beibringen der genannten Verletzung wurde die Lösung in einer Menge von 2,5 ml innerhalb von 2 Min. eingespritzt. Danach wird die verletzte Stelle von einer Gipshülle umschlossen oder mit einer Kunststoff-Binde umwickelt. Der Verlauf der Knochengeweberegeneration wird gemäß der Beschrei-bung aus dem Beispiel 2 überwacht. Der Zeitpunkt der Knochengeweberegeneration bis auf +0,5 Tage wird visuell nach dem Verhalten des verletzten Tieres bestimmt (ab dem Zeitpunkt der Lösungseinspritzung bis zum Zeitpunkt, wo die Stütz- und Bewegungsfunktion der verletzten Extremität völlig wiederhergestellt ist). Dieser Parameter ist für diesen spezifischen Fall der Tabelle 3 zu entnehmen.

### Beispiel 31

Die Vorgehensweise ist mit dem allgemeinen Beispiel 30 identisch, aber als Injektionslösung wird eine Wasserlösung verwendet, deren Zusammensetzung und Zubereitungstechnik vor der Injektion im Beispiel 4 beschrieben ist.

### Beispiel 32 (Vergleichsbeispiel)

Die Vorgehensweise ist mit dem allgemeinen Beispiel 30 identisch, aber als Injektionslösung wird eine Wasserlösung verwendet, deren Zusammensetzung und Zubereitungstechnik vor der Injektion im Beispiel 10 beschrieben ist.

### Beispiel 33 (Vergleichsbeispiel)

Die Vorgehensweise ist mit dem allgemeinen Beispiel 30 identisch, aber als Injektionslösung wird eine Wasserlösung verwendet, deren Zusammensetzung und Zubereitungstechnik vor der Injektion im Beispiel 11 beschrieben ist.

### Beispiel 34 (nach Prototyp [2])

Die Vorgehensweise ist mit dem allgemeinen Beispiel 30 identisch, aber als Injektionslösung wird eine Wasserlösung verwendet, deren Zusammensetzung und Zubereitungstechnik vor der Injektion im Beispiel 14 beschrieben ist.

### Beispiel 35 (nach Prototyp [2])

Die Vorgehensweise ist mit dem allgemeinen Beispiel 30 identisch, aber als Injektionslösung wird eine Wasserlösung verwendet, deren Zusammensetzung und Zubereitungstechnik vor der Injektion im Beispiel 15 beschrieben ist.

Die Zeitangaben hinsichtlich der Knochengeweberegeneration bei Anwendung der Verfahren gemäß den Beispielen 30 bis 35 sind in der Tabelle 3 enthalten.

**Tabelle 3**

| Beispiel Nr. | Verletztes Tier | Zeit bis zur vollständigen Instandsetzung der Stütz- und Bewegungsfunktion des verletzten Knochensegments, Tage |
|---|---|---|
| 30 | Hofhund unbestimmter Rasse | 7,5 |
| 31 | Hofhund unbestimmter Rasse | 8,0 |
| 32 (Vergleichsbeispiel) | Hofhund unbestimmter Rasse | 9,0 |
| 33 (Vergleichsbeispiel) | Hofhund unbestimmter Rasse | 8,0 |
| 34 (Prototyp) | Hofhund unbestimmter Rasse | 10,5 |

Aus den Daten der Tabellen 1 - 3 geht ganz klar hervor, dass die Anwendung des erfindungsgemäßen Verfahrens im Vergleich zum Prototyp-Verfahren [2] ermöglicht, die Dauer der Knochengeweberegeneration an der Verletzungsstelle wesentlich (um 20 - 25 %) zu verkürzen. Dabei liegt die genannte Verkürzung unabhängig von der Art der betroffenen Tiere vor. Gleichzeitig sei bemerkt, dass der verwendete Temperaturbereich von 30 - 100° C sowie die Temperierungszeit von 1 - 48 Stunden bei einer beliebigen Temperatur aus diesem Bereich sowie der pH-Wert der Lösung von 7.3 - 7.8 relevant sind. Die Unterschreitung sowie die Überschreitung dieser Bereiche werden in der Regel von der Verlängerung der Knochengewebe-regeneration begleitet (wird die Haltezeit bei genannter Temperatur verlängert, so werden die vorher erreichten Kennwerte praktisch gar nicht verändert, so dass die Verlängerung der Zeit über 48 Stunden schon belanglos ist). Es sei auch bemerkt, dass die durchgeführten Messungen der Festigkeit der Knochengewebe unter Einsatz der bekannten Verfahren [1, 2] und unter Einsatz des erfindungsgemäßen Verfahrens zur Knochengeweberegeneration keine merklichen Unterschiede ergaben. Die Angaben des klinischen Blutbilds während des Regenerationsvorgangs des verletzten Knochengewebes sowie die Beobachtungen des subjektiven Zustands der verletzten Tiere während ihrer Behandlung (Appetit, Reaktionsfähigkeit usw.) zeigen, dass die beim erfindungsgemäßen Verfahren benutzte Injektionslösung nicht die geringste ausgeprägte Giftigkeit aufweist; darüber hinaus ist keine der Zutaten laut [3, 4] dieser Lösung toxisch. Schließlich sei auch betont, dass die in der Erfindung verwendete Lösung gut haltbar ist und lange (mindestens 1 Jahr lang) in einem geschlossenen Behälter aufbewahrt werden kann, ohne dass ihre Wirksamkeit beeinträchtigt ist.

### LITERATURNACHWEIS

1. Patent RU 2061402. Verfahren zur Knochengeweberegeneration im Rahmen eines Versuchs / Devyatov F.V., Kholmogortsev E.A., Latypov A.L. // Infoblatt. Erfindungen, Heft 16, Veröff. 10.06.96.
2. Patent RU 2248210. Verfahren zur Knochengeweberegeneration im Rahmen eines Versuchs / Devyatov F.V., Kholmogortsev E.A. // Infoblatt. Erfindungen, Heft 8, Veröff. 20.03.05 (PROTOTYP)
3. Schädliche chemische Stoffe. Anorganische Verbindungen der I. bis IV. Gruppe / Herausgegeben von V.A. Filova. L.: Chimiya, 1988. SS. 111, 248-263.
4. N.M. Dyatlova, V.Y. Temkina, K.I. Popov. Metallkomplexone und -komplexonate. M.: Chimiya, 1988. S. 496-501.

## Patentansprüche

1. Wasserlösung enthaltend
| | |
|---|---|
| 1-Hydroxyethyliden-Diphosphonsäure in einer Menge von | (1,80-2,06) g/l, |
| wasserfreies Calciumchlorid in einer Menge von | (1,44-2,22) g/l, |
| Gadoliniumnitrat (III) in einer Menge von | (0,30-0,40) g/l, |
| Dysprosiumchlorid (III) Hexahydrat in einer Menge von | (0,038-0,076) g/l, |
| mit einem pH-Wert (7,3-7,8), | |
zur Verwendung in einem Verfahren zur Knochengeweberegeneration, in dem Bruchstücke eines verletzten Knochens zuerst mit einem Gipsverband immobilisiert werden, und anschließend die Wasserlösung in den Bruchbereich eingeführt wird, **dadurch gekennzeichnet**
**dass**, um die Dauer der Knochengewebegeneration an der Knochenverletzungs- oder Defektstelle sowie die Erholungszeit, bis die normale physiologische Funktion des verletzten/betroffenen Knochens wiederhergestellt ist, zu verkürzen,
die genannte Lösung vor der Einspritzung in den Bruchbereich bis zu einer Temperatur von (30-100)°C erwärmt, bei dieser Temperatur im Laufe von (1-48) Stunden gehalten und dann wieder auf Raumtemperatur abgekühlt wird.

## Claims

1. Water solution containing
| | |
|---|---|
| 1-hydroxyethylidene-diphosphonic acid in an amount of | (1.80 to 2.06) g/l, |
| anhydrous calcium chloride in an amount of | (1.44-2,22) g/l, |
| gadolinium nitrate (III) in an amount of | (0.30 to 0.40) g/l, |
| dysprosium (III) hexahydrate in an amount of | (0.038 to 0.076) g/l, |
| having a pH value | (7.3-7.8), |
for use in a method for bone tissue regeneration, in which the injured bone fragments are immobilized in a first plaster cast, and then the water solution is introduced into the fracture area,
**characterized in that**
prior to injection into the fracture area, the solution is heated up to a temperature of 30 to 100 °C and held at this temperature for a period of (1-48) hours and then cooled again to room temperature,
wherein this is intended to shorten the duration of bone tissue generation at the bone fracture or defect location, as well as the healing time until the normal physiological function of the injured/affected bone is restored.

## Revendications

1. Solution aqueuse contenant
de l'acide 1-hydroxyéthylidène-diphosphonique en une quantité de (1,80-2,06) g/l, du chlorure de calcium anhydre en une quantité de (1,44-2,22) g/l,
du nitrate de gadolinium (III) en une quantité de (0,30-0,40) g/l,
du chlorure de dysprosium (III) hexahydraté en une quantité de (0,038-0,076) g/l, avec un pH de (7,3-7,8),
pour une utilisation dans un procédé de régénération de tissu osseux, dans lequel des fragments d'un os lésé sont d'abord immobilisés à l'aide d'un plâtre, puis la solution aqueuse est introduite dans la zone de la fracture,
**caractérisé en ce que**, pour raccourcir la durée de la régénération du tissu osseux au niveau de la zone de lésion osseuse ou du défaut, ainsi que la durée de convalescence jusqu'à rétablissement de la fonction physiologique normale de l'os lésé/considéré, on chauffe jusqu'à une température de (30-100)°C la solution mentionnée, avant injection dans la zone de la fracture, on reste à cette température pendant (1-48) heures, puis on refroidit de nouveau à la température ambiante.
